# EUROPEAN PATENT SPECIFICATION

(11) **EP 1 282 710 B1**
(45) Date of publication and mention of the grant of the patent: **05.03.2008**
(21) Application number: 01911869.4
(22) Date of filing: 12.03.2001
(51) Int. Cl.: C12N 15/31, C07K 14/29, A61K 39/02, A61K 48/00, A61P 31/04

(54) **FISH VACCINE AGAINST PISCIRICKETTSIA SALMONIS**
FISCHIMPSTOFF GEGEN PISCIRICKETTSIA SALMONIS
VACCIN CONTRE LA PISCIRICKETTSIA SALMONIS DESTINE AUX POISSONS

(30) Priority: 11.03.2000 GB 0005838; 01.07.2000 GB 0016080; 01.07.2000 GB 0016082; 29.07.2000 GB 0018599
(43) Date of publication of application: 12.02.2003
(73) Proprietor: Novartis AG, 4056 Basel (CH); Novartis Pharma GmbH, 1230 Wien (AT)
(72) Inventor: SIMARD, Nathalie, Fredericton, New Brunswick E3B 6Z9 (CA); BROUWERS, Huub, Charlottetown, PEI C1A 4P3 (CA); JONES, Simon, Fisheries & Oceans, Vancouver, British Columbia V6B 5G3 (CA); GRIFFITHS, Steve, Fredericton, New Brunswick E3B 6Z9 (CA); VALENZUELA, Pablo, Santiago (CL); BURZIO, Luis, Santiago (CL)
(74) Representative: Gros, Florent
(86) International application number: PCT/GB2001/001055
(87) International publication number: WO 2001/068865

(56) References cited:
- JONES S R ET AL: "Virulence and antigenic characteristics of a cultured Rickettsiales-like organism isolated from farmed Atlantic salmon Salmo salar in eastern Canada." DISEASES OF AQUATIC ORGANISMS, (1998 MAY 14) 33 (1) 25-31. , XP001029879
- BARNES M N ET AL: "Purification of Piscirickettsia salmonis and partial characterization of antigens." DISEASES OF AQUATIC ORGANISMS, (1998 MAY 14) 33 (1) 33-41. , XP001029898
- MARSHALL S ET AL: "Minimally invasive detection of Piscirickettsia salmonis in cultivated salmonids via the PCR." APPLIED AND ENVIRONMENTAL MICROBIOLOGY, (1998 AUG) 64 (8) 3066-9. , XP001029515
- KUZYK M A ET AL: "OspA, a lipoprotein antigen of the obligate intracellular bacterial pathogen Piscirickettsia salmonis." JOURNAL OF MOLECULAR MICROBIOLOGY AND BIOTECHNOLOGY, (2001 JAN) 3 (1) 83-93. , XP001029507
- Kuzyk M.A. et al: (1996) Infection and Immunity 64:5205-5210
- Leong J.C. et al: (1997) Fish Vaccinology. Dev. Biol. Stand. 90:267-277
- Fryer J.L. et al: (1990) Fish Pathology 25:107-114

### SEQUENCE LISTING

<110> Novartis AG
   Simard, Nathalie
   Griffiths, Steve
   Brouwers, Huub
   Jones, Simon
   Valenzulela, Pablo
   Burzio, Luis
<120> sequence
<130> H-32319
<140> 01 911 869.4
   <141>
<150> PCT/GB01/01055
   <151> 2001-03-12.
<150> GB0005838.8
   <151> 2000-03-11
<150> GB0016080.4
   <151> 2000-07-01
<150> GB0016082.0
   <151> 2000-07-01
<150> GB0018599.1
   <151> 2000-07-29
<160> 30
<170> FastSEQ for windows version 4.0
<210> 1
   <211> 404
   <212> DNA
   <213> Piscirickettsia salmonis
<400> 1
   tgagtggcga ggagataagc taaaatgagt caagcattag ttgaatttaa aacggtagat 60
   catgtgctgc aagccttagg cgttgaaggg atgagtgcag cggatgtgca tggcctgctg 120
   attggtatgt tggcgagtca aagtaattta acctgtaaat catggttaga aaaagcgata 180
   tttatgggag ctcaccttga tgctgaaagt gatcttttta gtaatatcat ggccaaagag 240
   cagttaaagc agttagaggc attgtttaaa gtaagttggg agcagctctc tgcgggtgat 300
   tttacttttg ctctgctgtt acctgatggt aatgctgcgt taactgagcg tgcgagttta 360
   ttatgtgcct ggactcaagg ctttttaact ggcttgcatt tatc                  404
<210> 2
   <211> 126
   <212> PRT
   <213> Piscirickettsia salmonis
<400> 2
   Met Ser Gln Ala Leu Val Glu Phe Lys Thr Val Asp His Val Leu Gln
   1               5                   10                  15
   Ala Leu Gly Val Glu Gly Met Ser Ala Ala Asp Val His Gly Leu Leu
               20                  25                  30
   Ile Gly Met Leu Ala Ser Gln Ser Asn Leu Thr Cys Lys Ser Trp Leu
           35                  40                  45
   Glu Lys Ala Ile Phe Met Gly Ala His Leu Asp Ala Glu Ser Asp Leu
       50                  55                  60
   Phe Ser Asn Ile Met Ala Lys Glu Gln Leu Lys Gln Leu Glu Ala Leu
   65                  70                  75                  80
   Phe Lys Val Ser Trp Glu Gln Leu Ser Ala Gly Asp Phe Thr Phe Ala
                   85                  90                  95
   Leu Leu Leu Pro Asp Gly Asn Ala Ala Leu Thr Glu Arg Ala Ser Leu
               100                 105                 110
   Leu Cys Ala Trp Thr Gln Gly Phe Leu Thr Gly Leu His Leu
           115                 120                 125
<210> 3
   <211> 591
   <212> DNA
   <213> Piscirickettsia salmonis
<400> 3
   atgagtcaag cattagttga atttaaaacg gtagatcatg tgctgcaagc cttaggcgtt 60
   gaagggatga gtgcagcgga tgtgcatggc ctgctgattg gtatgttggc gagtcaaagt 120
   aatttaacct gtaaatcatg gttagaaaaa gcgatattta tgggagctca ccttgatgct 180
   gaaagtgatc tttttagtaa tatcatggcc aaagagcagt taaagcagtt agaggcattg 240
   tttaaagtaa gttgggagca gctctctgcg ggtgatttta cttttgctct gctgttacct 300
   gatggtaatg ctgcgttaac tgagcgtgcg agtttattat gtgcctggac tcaaggcttt 360
   ttaactggct tgcatttatc gggtgttaat attgctaaat ataaagaagg tgaattagcg 420
   acgaccttaa aagatttagc tgaagttgcg cagttggatt tagccattga agacagtaat 480
   gaaaatgaag cggcatatac tgagattgct gaatatgtac gtatggcggc gctttttgtt 540
   catagtgaat tagccggttc tggtcaagcg actcagatga ctgttcatta a          591
<210> 4
   <211> 196
   <212> PRT
   <213> Piscirickettsia salmonis
<400> 4
   Met Ser Gln Ala Leu Val Glu Phe Lys Thr Val Asp His Val Leu Gln
    1               5                  10                  15
   Ala Leu Gly Val Glu Gly Met Ser Ala Ala Asp Val His Gly Leu Leu
               20                  25                  30
   Ile Gly Met Leu Ala Ser Gln Ser Asn Leu Thr Cys Lys Ser Trp Leu
           35                  40                  45
   Glu Lys Ala Ile Phe Met Gly Ala His Leu Asp Ala Glu Ser Asp Leu
       50                  55                  60
   Phe Ser Asn Ile Met Ala Lys Glu Gln Leu Lys Gln Leu Glu Ala Leu
   65                  70                  75                  80
   Phe Lys Val Ser Trp Glu Gln Leu Ser Ala Gly Asp Phe Thr Phe Ala
                   85                  90                  95
   Leu Leu Leu Pro Asp Gly Asn Ala Ala Leu Thr Glu Arg Ala Ser Leu
               100                 105                 110
   Leu Cys Ala Trp Thr Gln Gly Phe Leu Thr Gly Leu His Leu Ser Gly
           115                 120                 125
   Val Asn Ile Ala Lys Tyr Lys Glu Gly Glu Leu Ala Thr Thr Leu Lys
       130                 135                 140
   Asp Leu Ala Glu Val Ala Gln Leu Asp Leu Ala Ile Glu Asp Ser Asn
   145                 150                 155                 160
   Glu Asn Glu Ala Ala Tyr Thr Glu Ile Ala Glu Tyr Val Arg Met Ala
                   165                 170                 175
   Ala Leu Phe Val His Ser Glu Leu Ala Gly Ser Gly Gln Ala Thr Gln
               180                 185                 190
   Met Thr Val His
           195
<210> 5
   <211> 489
   <212> DNA
   <213> Piscirickettsia salmonis
<400> 5
   atgaacagag gatgtttgca aggtagtagt ctaattatta tcagtgtgtt tttagttggc 60
   tgtgcccaga actttagtcg tcaagaagtc ggagctgcga ctggggctgt tgttggcggt 120
   gttgctggcc agctgtttgg taaaggtagt ggtcgagttg caatggccat tggtggtgct 180
   gttttgggtg gattaattgg ttctaaaatc ggtcaatcga tggatcagca ggataaaata 240
   aagctaaacc agagtttgga aaaggtaaaa gcagggcaag tgacacgttg gcgtaatcca 300
   gatacaggca atagttatag tgttgagcca gtgcgtactt accagcgtta caataagcaa 360
   gagcgtcgcc agcaatattg tcgagaattt cagcaaaagg cgatgattgc agggcagaag 420
   caagagattt acggcactgc atgccggcaa ccggatggtc gttggcaagt catttcaaca 480
   gaaaaataa                                                         489
<210> 6
   <211> 162
   <212> PRT
   <213> Piscirickettsia salmonis
<400> 6
   Met Asn Arg Gly Cys Leu Gln Gly Ser Ser Leu Ile Ile Ile Ser Val
   1 5 10 15
   Phe Leu Val Gly Cys Ala Gln Asn Phe Ser Arg Gln Glu Val Gly Ala
   20 25 30
   Ala Thr Gly Ala Val Val Gly Gly Val Ala Gly Gln Leu Phe Gly Lys
   35 40 45
   Gly Ser Gly Arg Val Ala Met Ala Ile Gly Gly Ala Val Leu Gly Gly
   50 55 60
   Leu Ile Gly Ser Lys Ile Gly Gln Ser Met Asp Gln Gln Asp Lys Ile
   65 70 75 80
   Lys Leu Asn Gln Ser Leu Glu Lys Val Lys Ala Gly Gln Val Thr Arg
   85 90 95
   Trp Arg Asn Pro Asp Thr Gly Asn Ser Tyr Ser Val Glu Pro Val Arg
   100 105 110
   Thr Tyr Gln Arg Tyr Asn Lys Gln Glu Arg Arg Gln Gln Tyr Cys Arg
   115 120 125
   Glu Phe Gln Gln Lys Ala Met Ile Ala Gly Gln Lys Gln Glu Ile Tyr
   130 135 140
   Gly Thr Ala Cys Arg Gln Pro Asp Gly Arg Trp Gln Val Ile Ser Thr
   145 150 155 160
   Glu Lys
<210> 7
   <211> 1098
   <212> DNA
   <213> Piscirickettsia salmonis
<220>
   <221> misc_feature
   <222> 765, 1064
   <223> n = A,T,C or G
<400> 7
   caaggaatat tggcaaaatg ggagaatgtt tctgctcaaa aagtgatggt tgccaaggtt 60
   agtacagtta ataatgctgg agataatggt cagtcaggta aaaataataa tgaaaatatt 120
   attgaaaaag caggtgctat tgtatttgcg gtactcgata cacagctaaa cagtgaccaa 180
   cccgggactc cagtaatggc aacgattgtt caaggtaaat ttaaaaatgc caaattgttg 240
   ggtagcttta aaagagagga tgaaaaacta gtcatttctt ttgatcgcat atctttgcct 300
   gaacttgatc acagtatttc tattaaggcg tatgcaatta atgccacaac agcacaaaat 360
   gcactgtctt cagatgtaga taatcattat ttattacgtt atggtgggct ctttgctgct 420
   gcgtttttgc aaggctttgg cgattatttc tcccaaaact catcaagctt atgtggtggt 480
   gcgacaacct gtattattac aggcactcaa tcaactgcag aacaaaatcg tacaacgaga 540
   aaagccctat attctggttt aggtcaagtt ggaacaactt tagctggtaa agcaagcgct 600
   gcatttgatc gccctccaac ggttacttta aatcaaggtg ttggtatggg gattttattt 660
   atgtcggatg taaaggtgta agttaaaatg agtaataatc acacagataa aaattataat 720
   tttgatgatg atcaagacat taatgaggat aaagaagacc ttgcngctag ataatgagcc 780
   agcagttaaa agaaataatg cagatgctgt ttggcaaggg acttcactgt gggataaaat 840
   aaggccgatg ttgcattatt atatcattgc tattattgca ttcgctgtag caggttatat 900
   gatgtataac gcataccgaa ctttatatcc aaagcagtca gtgcagcagg ctgaggctaa 960
   ccatttaagc tttagtaatc aggttgaaac tggcagtaag tcagcgaaag gcttttctcc 1020
   cctggctcag tctcaagaaa ataaggtcaa aaataaaagt gggncttgga aaaaaagaag 1080
   agataaaacc gaatgcta 1098
<210> 8
   <211> 226
   <212> PRT
   <213> Piscirickettsia salmonis
<400> 8
   Gln Gly Ile Leu Ala Lys Trp Glu Asn Val Ser Ala Gln Lys Val Met
   1               5                   10                  15
   Val Ala Lys Val Ser Thr Val Asn Asn Ala Gly Asp Asn Gly Gln Ser
               20                  25                  30
   Gly Lys Asn Asn Asn Glu Asn Ile Ile Glu Lys Ala Gly Ala Ile Val
           35                  40                  45
   Phe Ala Val Leu Asp Thr Gln Leu Asn Ser Asp Gln Pro Gly Thr Pro
       50                  55                  60
   Val Met Ala Thr Ile Val Gln Gly Lys Phe Lys Asn Ala Lys Leu Leu
   65                  70                  75                  80
   Gly Ser Phe Lys Arg Glu Asp Glu Lys Leu Val Ile Ser Phe Asp Arg
                   85                  90                  95
   Ile Ser Leu Pro Glu Leu Asp His Ser Ile Ser Ile Lys Ala Tyr Ala
               100                 105                 110
   Ile Asn Ala Thr Thr Ala Gln Asn Ala Leu Ser Ser Asp Val Asp Asn
           115                 120                 125
   His Tyr Leu Leu Arg Tyr Gly Gly Leu Phe Ala Ala Ala Phe Leu Gln
       130                 135                 140
   Gly Phe Gly Asp Tyr Phe Ser Gln Asn Ser Ser Ser Leu Cys Gly Gly
   145                 150                 155                 160
   Ala Thr Thr Cys Ile Ile Thr Gly Thr Gln Ser Thr Ala Glu Gln Asn
                   165                 170                 175
   Arg Thr Thr Arg Lys Ala Leu Tyr Ser Gly Leu Gly Gln Val Gly Thr
               180                 185                 190
   Thr Leu Ala Gly Lys Ala Ser Ala Ala Phe Asp Arg Pro Pro Thr Val
           195                 200                 205
   Thr Leu Asn Gln Gly Val Gly Met Gly Ile Leu Phe Met Ser Asp Val
       210                 215                 220
   Lys Val
   225
<210> 9
   <211> 30
   <212> PRT
   <213> Piscirickettsia salmonis
<400> 9
   Ala Asp Asn Gly Lys Leu Gln Leu Gln Ile Ser Gln Leu Lys Ala Gln
   1               5                   10                  15
   Gln Thr Gln Leu Gln Gln Gln Val Ala Asn Leu Gln Gly Gln
               20                  25                  30
<210> 10
   <211> 164
   <212> DNA
   <213> Piscirickettsia salmonis
<400> 10
   gatctttaat ctcattaaaa tttatgtttc taatcgcgtg ggttattttc atcaaatacg 60
   gcatttttag gaggagggat aacaacatca gcattaggtg agtggtttaa aacggaatcg 120
   taaacatcat ggctatcgta ggctccgtct gcagtgaagc gatc                  164
<210> 11
   <211> 44
   <212> PRT
   <213> Piscirickettsia salmonis
<400> 11
   Asp Arg Phe Thr Ala Asp Gly Ala Tyr Asp Ser His Asp Val Tyr Asp
    1               5                  10                  15
   Ser Val Leu Asn His Ser Pro Asn Ala Asp Val Val Ile Pro Pro Pro
               20                  25                  30
   Lys Asn Ala Val Phe Asp Glu Asn Asn Pro Arg Asp
           35                  40
<210> 12
   <211> 195
   <212> DNA
   <213> Piscirickettsia salmonis
<220>
   <221> misc_feature
   <222> 31, 41, 147
   <223> n = A,T,C or G
<400> 12
   ataagcctag atgatgatat tgcgggaata nccattgatt nacaggcctt aagcgttttg 60
   gccgtgacga tgtggcacca agaaaaatac aagatatcag caaagcgcag ctggcgtaaa 120
   cttcatgtgg ccgttgatga tgatcantat attcaagccg cactcatcac cgatcgctat 180
   gaagcagatg aggag                                                  195
<210> 13
   <211> 65
   <212> PRT
   <213> Piscirickettsia salmonis
<220>
   <221> VARIANT
   <222> 11, 14, 49
   <223> Xaa = Any Amino Acid
<400> 13
   Ile Ser Leu Asp Asp Asp Ile Ala Gly Ile Xaa Ile Asp Xaa Gln Ala
    1               5                  10                  15
   Leu Ser Val Leu Ala Val Thr Met Trp His Gln Glu Lys Tyr Lys Ile
               20                  25                  30
   Ser Ala Lys Arg Ser Trp Arg Lys Leu His Val Ala Val Asp Asp Asp
           35                  40                  45
   Xaa Tyr Ile Gln Ala Ala Leu Ile Thr Asp Arg Tyr Glu Ala Asp Glu
       50                  55                  60
   Glu
   65
<210> 14
   <211> 213
   <212> DNA
   <213> Piscirickettsia salmonis
<220>
   <221> misc_feature
   <222> 186
   <223> n = A,T,C or G
<400> 14
   tatgagatta aagatcatgg tagaatgcac tggcaaaaga cacgacaata cggcaagcgt 60
   aattattctg agttggcgat tcagcgttac aaacgcattt tgggcaacac gatgcagtcc 120
   agagacatat cgcgacagaa aaatgaagga ctaattggcg cgggtatttt aaatagagat 180
   gaccantctc ggcatgccgg tgacaataat gta                              213
<210> 15
   <211> 71
   <212> PRT
   <213> Piscirickettsia salmonis
<220>
   <221> VARIANT
   <222> 62
   <223> Xaa = Any Amino Acid
<400> 15
   Tyr Glu Ile Lys Asp His Gly Arg Met His Trp Gln Lys Thr Arg Gln
    1               5                  10                  15
   Tyr Gly Lys Arg Asn Tyr Ser Glu Leu Ala Ile Gln Arg Tyr Lys Arg
               20                  25                  30
   Ile Leu Gly Asn Thr Met Gln Ser Arg Asp Ile Ser Arg Gln Lys Asn
           35                  40                  45
   Glu Gly Leu Ile Gly Ala Gly Ile Leu Asn Arg Asp Asp Xaa Ser Arg
       50                  55                  60
   His Ala Gly Asp Asn Asn Val
   65                  70
<210> 16
   <211> 115
   <212> DNA
   <213> Piscirickettsia salmonis
<220>
   <221> misc_feature
   <222> 7
   <223> n = A,T,C or G
<400> 16
   gatcaangcc cgcatattaa tcgacgacca cgatattcaa aagttaaaaa ttcaaaatat 60
   ccgccaacat attgcctatt tacctcagca tggtgactta tttaatggca cgatc      115
<210> 17
   <211> 38
   <212> PRT
   <213> Piscirickettsia salmonis
<220>
   <221> VARIANT
   <222> 2
   <223> Xaa = Any Amino Acid
<400> 17
   Ile Xaa Ala Arg Ile Leu Ile Asp Asp His Asp Ile Gln Lys Leu Lys
    1               5                  10                  15
   Ile Gln Asn Ile Arg Gln His Ile Ala Tyr Leu Pro Gln His Gly Asp
               20                  25                  30
   Leu Phe Asn Gly Thr Ile
           35
<210> 18
   <211> 179
   <212> DNA
   <213> Piscirickettsia salmonis
<220>
   <221> misc_feature
   <222> 25, 141, 164, 167, 177
   <223> n = A,T,C or G
<400> 18
   gcacgcatat cgatacctaa cgtcntttca agaacaaaaa tattataaac aagccataga 60
   agtcagccaa ttacttggcc ttgactcaat tattgagcgc ttgcccaaag gctatcacac 120
   tcctgttgcc aatcatgccg natattaatt gactacgcac gatnttncaa agcttantg  179
<210> 19
   <211> 48
   <212> PRT
   <213> Piscirickettsia salmonis
<220>
   <221> VARIANT
   <222> 47
   <223> Xaa = Any Amino Acid
<400> 19
   His Ala Tyr Arg Tyr Leu Thr Ser Phe Gln Glu Gln Lys Tyr Tyr Lys
    1               5                  10                  15
   Gln Ala Ile Glu Val Ser Gln Leu Leu Gly Leu Asp Ser Ile Ile Glu
               20                  25                  30
   Arg Leu Pro Lys Gly Tyr His Thr Pro Val Ala Asn His Ala Xaa Thr
           35                  40                  45
<210> 20
   <211> 390
   <212> DNA
   <213> Piscirickettsia salmonis
<220>
   <221> misc_feature
   <222> 55
   <223> n = A,T,C or G
<400> 20
   ttattgagcg cttgcccaaa ggctatcaca ctcctgttgc caatcatgcc atggnagtcg 60
   ctacctcgcg gtatcattca gcgcattgcg attgcccgtg ccctgattca taagccacca 120
   atcgtcctat tcgatgaggc caatacggcc atggacatgc aaggtgatac catcttaatt 180
   aatgtgcttg aacaacttaa aggcacctgc acactcatcc tcgtctctca tcgcccatca 240
   ttgctggcac atgcagataa aatctttatc ctcgagaata aaaatctggt ggagaaagtc 300
   acatgagctc tgcactaacc gcccaggagc ataatattcg cactgcgttt attaacagcc 360
   tcgaaccact gttaactgca ttaggctggc                                  390
<210> 21
   <211> 101
   <212> PRT
   <213> Piscirickettsia salmonis
<220>
   <221> VARIANT
   <222> 19
   <223> Xaa = Any Amino Acid
<400> 21
   Leu Leu Ser Ala Cys Pro Lys Ala Ile Thr Leu Leu Leu Pro Ile Met
    1               5                  10                  15
   Pro Trp Xaa Ser Leu Pro Arg Gly Ile Ile Gln Arg Ile Ala Ile Ala
               20                  25                  30
   Arg Ala Leu Ile His Lys Pro Pro Ile Val Leu Phe Asp Glu Ala Asn
           35                  40                  45
   Thr Ala Met Asp Met Gln Gly Asp Thr Ile Leu Ile Asn Val Leu Glu
       50                  55                  60
   Gln Leu Lys Gly Thr Cys Thr Leu Ile Leu Val Ser His Arg Pro Ser
   65                  70                  75                  80
   Leu Leu Ala His Ala Asp Lys Ile Phe Ile Leu Glu Asn Lys Asn Leu
                   85                  90                  95
   Val Glu Lys Val Thr
               100
<210> 22
   <211> 280
   <212> DNA
   <213> Piscirickettsia salmonis
<220>
   <221> misc_feature
   <222> 9, 38, 236, 255, 256, 273
   <223> n = A,T,C or G
<400> 22
   ggttgttgnt ggaatatcaa ggctaatatt ggtaaatngc caattttcat catcttcatt 60
   tgtgcttgta ttattatttt tatcataacg aaaattgaca tgttcaaagc aaatgtggcc 120
   atcaatatgg gcgagttgct tttgcgtggg aacatactct ggagggagtt taaagacttc 180
   ttgtacattt atcttcagca ctaccaatcg actgtaagcg gttccagacg gaggtngcac 240
   gattaattgg ctgtnnacag cgccctgaca gtnacggtgc                       280
<210> 23
   <211> 91
   <212> PRT
   <213> Piscirickettsia salmonis
<220>
   <221> VARIANT
   <222> 3, 9, 15, 81, 91
   <223> Xaa = Any Amino Acid
<400> 23
   His Arg Xaa Cys Gln Gly Ala Val Xaa Ser Gln Leu Ile Val Xaa Pro
    1               5                  10                  15
   Pro Ser Gly Thr Ala Tyr Ser Arg Leu Val Val Leu Lys Ile Asn Val
               20                  25                  30
   Gln Glu Val Phe Lys Leu Pro Pro Glu Tyr Val Pro Thr Gln Lys Gln
           35                  40                  45
   Leu Ala His Ile Asp Gly His Ile Cys Phe Glu His Val Asn Phe Arg
       50                  55                  60
   Tyr Asp Lys Asn Asn Asn Thr Ser Thr Asn Glu Asp Asp Glu Asn Trp
   65                  70                  75                  80
   Xaa Phe Thr Asn Ile Ser Leu Asp Ile Pro Xaa
                   85                  90
<210> 24
   <211> 170
   <212> DNA
   <213> Piscirickettsia salmonis
<400> 24
   ggatcatgct catcctctac gtcgatgcga tggtctcacc tttaggaacg gctttagcct 60
   ataccggctc ttctacacgg atgctaacgg ccatgtctcg cgaaaaacag gttccgcgtt 120
   tctttgacca tgtacacccc cactatggtg tttcccgtcg ttcattgatc           170
<210> 25
   <211> 56
   <212> PRT
   <213> Piscirickettsia salmonis
<400> 25
   Ile Met Leu Ile Leu Tyr Val Asp Ala Met Val Ser Pro Leu Gly Thr
    1               5                  10                  15
   Ala Leu Ala Tyr Thr Gly Ser Ser Thr Arg Met Leu Thr Ala Met Ser
               20                  25                  30
   Arg Glu Lys Gln Val Pro Arg Phe Phe Asp His Val His Pro His Tyr
           35                  40                  45
   Gly Val Ser Arg Arg Ser Leu Ile
       50                  55
<210> 26
   <211> 199
   <212> DNA
   <213> Piscirickettsia salmonis
<400> 26
   gatgagcatg atcatatgca ggcctaatag accggctaac tgtaccatgg gtgcttgaaa 60
   gtcaagctgg tgccagccat ttttaagcat acttggcggt agtgcaccaa taaatgacac 120
   ttgacaagag caagtaaatc actaagcaga ttacaatcga gaggaccaga gagagcggga 180
   tattgcgttt tgggtttga                                              199
<210> 27
   <211> 38
   <212> PRT
   <213> Piscirickettsia salmonis
<400> 27
   Ser Asn Pro Lys Arg Asn Ile Pro Leu Ser Leu Val Leu Ser Ile Val
    1               5                  10                  15
   Ile Cys Leu Val Ile Tyr Leu Leu Leu Ser Ser Val Ile Tyr Trp Cys
               20                  25                  30
   Thr Thr Ala Lys Tyr Ala
           35
<210> 28
   <211> 375
   <212> DNA
   <213> Piscirickettsia salmonis
<220>
   <221> misc_feature
   <222> 7
   <223> n = A,T,C or G
<400> 28
   ggtaaangag tatcgatatt ggcgtttttt ggctgtattt tatggttcag gttgtgcgag 60
   tacggtgcca acagggcgcc ttattctgat tatcctcatg tgtatgcatg cccgaataag 120
   ttaagtactt tgtgttatcg tacagcgatt gcaccggttg gacactggtc tcagtataat 180
   cagctgagct ttcagttgcc gattgctttg caagtaccat tgcgtcaagg acaattagag 240
   ctacaagagt attatgctaa aaatcccgta ttgccttcat ctttgccttt atcaggccca 300
   ggcccgttaa cgtcttattt atatccattt ggattgtgtg caacaaaaat aattcgctta 360
   gagagtttaa ctgat                                                   375
<210> 29
   <211> 125
   <212> PRT
   <213> Piscirickettsia salmonis
<220>
   <221> VARIANT
   <222> 3
   <223> Xaa = Any Amino Acid
<400> 29
   Gly Lys Xaa Val Ser Ile Leu Ala Phe Phe Gly Cys Ile Leu Trp Phe
    1               5                  10                  15
   Arg Leu Cys Glu Tyr Gly Ala Asn Arg Ala Pro Tyr Ser Asp Tyr Pro
               20                  25                  30
   His Val Tyr Ala Cys Pro Asn Lys Leu Ser Thr Leu Cys Tyr Arg Thr
           35                  40                  45
   Ala Ile Ala Pro Val Gly His Trp Ser Gln Tyr Asn Gln Leu Ser Phe
       50                  55                  60
   Gln Leu Pro Ile Ala Leu Gln Val Pro Leu Arg Gln Gly Gln Leu Glu
   65                  70                  75                  80
   Leu Gln Glu Tyr Tyr Ala Lys Asn Pro Val Leu Pro Ser Ser Leu Pro
                   85                  90                  95
   Leu Ser Gly Pro Gly Pro Leu Thr Ser Tyr Leu Tyr Pro Phe Gly Leu
               100                 105                 110
   Cys Ala Thr Lys Ile Ile Arg Leu Glu Ser Leu Thr Asp
           115                 120                 125
<210> 30
   <211> 639
   <212> PRT
   <213> Piscirickettsia salmonis
<220>
   <221> VARIANT
   <222> 251, 252, 253, 254, 255, 256, 257, 258, 259, 260, 261, 262
   <223> Xaa = Any Amino Acid
<400> 30
   Met Ala Glu Ile Ile Gly Ile Asp Leu Gly Thr Thr Asn Ser Cys Val
   1               5                   10                  15
   Ala Val Leu Asp Gly Asp Lys Pro Arg Val Ile Glu Ser Ala Glu Gly
               20                  25                  30
   Asp Arg Thr Thr Pro Ser Ile Val Ala Tyr Thr Asn Asp Gly Val Thr
           35                  40                  45
   Val Gly Gln Pro Ala Lys Arg Gln Ala Val Thr Asn Pro Asn Asn Thr
       50                  55                  60
   Leu Phe Ala Val Lys Arg Leu Ile Gly Arg Lys Ser Ser Asp Asp Thr
   65                  70                  75                  80
   Val Gln Arg Asp Ile Glu Arg Leu Pro Tyr Thr Ile Ala Ala Ala Asp
                   85                  90                  95
   Asn Gly Asp Ala Trp Ile Asp Val Asn Gly Glu Lys Leu Ala Pro Pro
               100                 105                 110
   Gln Ile Ser Ala Gln Val Leu Ala Lys Met Lys Lys Thr Ala Glu Asp
           115                 120                 125
   Tyr Leu Gly Glu Asp Val Lys Glu Ala Val Ile Thr Val Pro Ala Tyr
       130                 135                 140
   Phe Asn Asp Ala Gln Arg Gln Ala Thr Lys Asp Ala Gly Arg Ile Ala
   145                 150                 155                 160
   Gly Leu Asp Val Lys Arg Ile Ile Asn Glu Pro Thr Ala Ala Ala Leu
                   165                 170                 175
   Ala Tyr Gly Met Asp Lys Lys Arg Gly Asp Gly Val Ile Ala Val Tyr
               180                 185                 190
   Asp Leu Gly Gly Gly Thr Phe Asp Ile Ser Ile Ile Glu Ile Ala Glu
           195                 200                 205
   Val Asp Gly Glu His Gln Phe Glu Val Leu Ala Thr Asn Gly Asp His
       210                 215                 220
   Thr Leu Gly Gly Glu Asp Phe Asp Leu Arg Leu Ile Ser Tyr Leu Val
   225                 230                 235                 240
   Asp Glu Phe Lys Lys Glu Gln Gly Ile Asp Xaa Xaa Xaa Xaa Xaa Xaa
                   245                 250                 255
   Xaa Xaa Xaa Xaa Xaa Xaa Glu Ala Ser Glu Lys Ala Lys Ile Glu Leu
               260                 265                 270
   Ser Ser Thr Gln Gln Thr Asp Val Asn Leu Pro Tyr Ile Thr Ala Asp
           275                 280                 285
   Ala Thr Gly Pro Lys His Met Asn Ile Arg Val Thr Arg Ala Lys Phe
       290                 295                 300
   Glu Ser Leu Val Glu Asp Leu Val Glu Gly Thr Ile Glu Pro Cys Arg
   305                 310                 315                 320
   Val Ala Leu Lys Asp Ala Gly Leu Ser Val Asn Asp Val Thr Asp Val
                   325                 330                 335
   Ile Leu Val Gly Gly Gln Thr Arg Met Pro Lys Ala Gln Ala Val Val
               340                 345                 350
   Lys Asn Phe Phe Gly Lys Glu Pro Arg Arg Asp Val Asn Pro Asp Glu
           355                 360                 365
   Ala Val Ala Val Gly Ala Ala Ile Gln Gly Gly Val Leu Ala Gly Asp
       370                 375                 380
   Val Lys Asp Val Met Leu Leu Asp Val Thr Pro Leu Ser Leu Gly Ile
   385                 390                 395                 400
   Glu Thr Met Gly Gly Val Met Thr Lys Leu Ile Glu Lys Asn Thr Thr
                   405                 410                 415
   Ile Pro Thr Lys Ser Gln Thr Phe Ser Thr Ala Gln Asp Asn Gln Asn
               420                 425                 430
   Ala Val Thr Val His Val Leu Gln Gly Glu Arg Glu Val Ala Thr Gly
           435                 440                 445
   Lys Lys Leu Thr Gly Arg Phe Asp Leu Ala Asp Ile Pro Pro Ala Pro
       450                 455                 460
   Arg Gly Met Pro Leu Ile Leu Arg Val His Phe Asp Ile Asp Ala Asn
   465                     470                     475                     480
   Gly Thr Leu Asn Val Ser Ala Lys Asp Lys Gly Thr Gly Lys Glu Gln
                   485                     490                     495
   Ser Ile Val Ile Arg Arg Ser Ser Gly Leu Ser Asp Asp Glu Val Asp
               500                     505                     510
   Ala Met Ile Lys Asp Ala Glu Asp His Ala Asp Asp Asp Lys Lys Phe
           515                     520                     525
   Gln Glu Leu Val Gly Ala Arg Asn Asn Ala Glu Ala Met Ile His Ala
       530                     535                     540
   Thr Glu Lys Gly Leu Lys Glu Ala Asp Gly Lys Val Ala Ala Asp Asp
   545                     550                     555                     560
   Lys Thr Ala Ile Glu Lys Ala Ile Ser Glu Leu Lys Asp Val Val Ser
                   565                     570                     575
   Gly Leu Asp Lys Ala Val Ile Asp Glu Lys Val Glu Ala Leu Thr Gln
               580                     585                     590
   Ala Ser Ala Lys Met Ala Glu Val Leu Tyr Ala Asn Gln Gly Ala Glu
           595                     600                     605
   Ala Glu Ala Ala Ala Ala Gly Ala Glu Gln Ala Gln Ser Gln Thr Asp
       610                     615                     620
   Glu Lys Lys Asp Asp Asp Val Val Asp Ala Glu Phe Glu Glu Val
   625                     630                     635

## Description

The present application describes a fish vaccine. More specifically a vaccine to protect salmon against piscirickettsiosis also referred to as salmonid rickettsial septicaemia (SRS).

It is recognized, for example, in Jones et al. (1998) Dis Aquat. Org: 33: 25-31, that there are antigenic similarities amongst strains of rickettsial-like organisms in salmonids. Marshall et al. (1998) App.Env. Micro, 64, 8, 3066-3069 also describes a screening assay for Piscirickettsia salmonis.

However, to date no commercially available vaccine is effective against Piscirickettsia salmonis, the causative agent of SRS. Accordingly there is a need for an effective vaccine against Piscirickettsia salmonis.

The present application describes a fish vaccine. More specifically a nucleic acid vaccine for administration to fish to protect against piscirickettsiosis also referred to as salmonid rickettsial septicaemia (SRS).

The application further describes an amino acid sequence for administration to fish to protect against SRS.

The present invention provides at least one nucleic acid sequence and / or an amino acid sequence which is derived from Piscirickettsia salmonis or a synthetically prepared analogue thereof or a substantially homologous sequence.

A substantially homologous nucleic acid sequence is a sequence which can be transcribed and/or translated to provide an amino acid sequence which is substantially homologous to at least a part of a surface antigen present on Piscirickettsia salmonis.

A substantially homologous amino acid sequence corresponds to at least 70% of a surface antigen and induces an immune response.

More preferably the homologous amino acid sequence corresponds to at least 90% of the amino acid sequence.

A typical nucleic acid sequence is chosen from the sequences of Psclone51A p10.6 (also known as immuno-reactive clone 0110-2-5), IcmE, clone3/original, clone3/3PST-R, clone3.3APA-F, clone7/original, clone7/XbaR, clone7/7MunR, clone7/7MunF, clone20/original, clone20/20VSPF or clone 15. Typically the amino acid sequence is derived from one of the above-mentioned nucleic acid sequences or is chosen from the sequences of p45 or HSP70 antigen.

Preferably peptide sequences or nucleic acid sequences are identified herein as sequence ID numbers 1,3,5,7,10,12,14,16,18,20,22,24,26 and 28.

The application describes the use of nucleic acid sequences or peptide sequence as defined herein in the preparation of a vaccine for the protection of fish against Piscirickettsia salmonis.

A vaccine to protect fish against Piscirickettsia salmonis wherein the vaccine includes nucleic acid or peptide sequences as defined herein.

### Background of the Invention

Barnes et al. (1998) Dis. Aquat. Org. 33, 33-41 and Kuzyk et al. (1996) Infect. And Immunity, 64, 12, 5205-5210 provide antigenic characterisation of some Piscirickettsia salmonis sequences, none of which have significant homology to the sequences of the present invention.

Table 1 provides the SEQ ID No. and the corresponding Figure number for the sequences of the present invention.

**Table 1: SEQ ID No and Figure Number**

| **SEQ ID No.** | **Figure** | **Description** |
|---|---|---|
| 1 | 1 | DNA sequence Psclone51 A |
| 2 | 1 | amino acid sequence for Psclone51 A |
| 3 | 2 | complete DNA sequence for Psclone51A |
| 4 | 3 | complete amino acid sequence for Psclone51A |
| 5 | 4 | nucleotide sequence for p10.6 |
| 6 | 5 | amino acid sequence for p10.6 |
| 7 | 6 | DNA sequence for IcmE |
| 8 | 6 | amino acid sequence for IcmE |
| 9 | 7 | amino acid sequence for p45 antigen |
| 10 | 8 | DNA sequence for clone3/original |
| 11 | 8 | amino acid sequence for clone3/original, in the 3' to 5' direction of the listed DNA strand |
| 12 | 9 | DNA sequence for clone3/3PSTR |
| 13 | 9 | amino acid sequence for clone3/3PSTR |
| 14 | 10 | DNA sequence for clone3/3APA-F |
| 15 | 10 | amino acid sequence for clone3/3APA-F |
| 16 | 11 | DNA sequence for clone7/original |
| 17 | 11 | amino acid sequence for clone7/original |
| 18 | 12 | DNA sequence for clone7/Xbar |
| 19 | 12 | amino acid sequence for clone7/Xbar |
| 20 | 13 | DNA sequence for clone7/MunR |
| 21 | 13 | amino acid sequence for clone7/MunR |
| 22 | 14 | DNA sequence for clone7/MunF |
| 23 | 14 | amino acid sequence for clone7/MunF, in the 3' to 5' direction of the listed DNA strand |
| 24 | 15 | DNA sequence for clone20/original |
| 25 | 15 | amino acid sequence for clone20/original |
| 26 | 16 | DNA sequence for clone 20/20VSPF |
| 27 | 16 | amino acid sequence for clone 20/20VSPF, in the 3' to 5' direction of the listed DNA strand |
| 28 | 17 | DNA sequence for clone 15/original |
| 29 | 17 | amino acid sequence for clone 15/original |
| 30 | 18 | amino acid sequence for Hsp70; |

### SEQ ID No. 1,2,3,4 - Psclone 51A

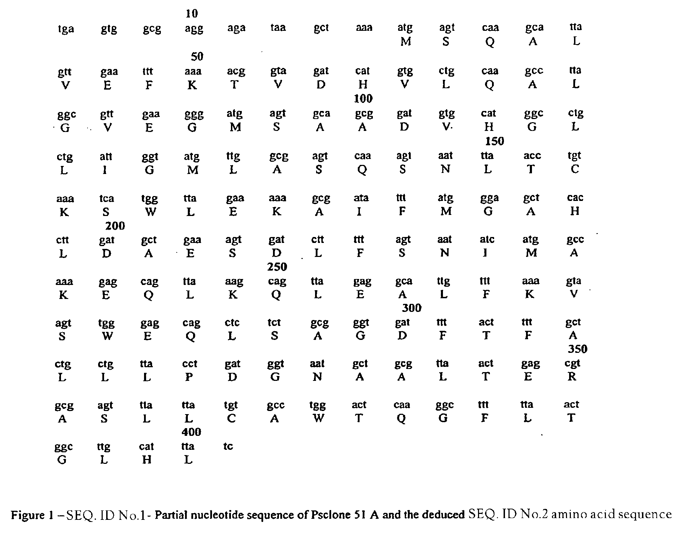

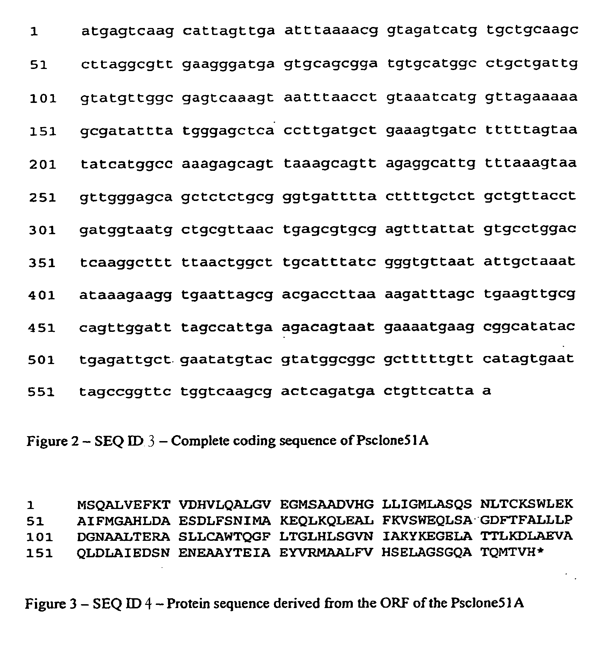

A detailed description of the invention, listing the exact nucleotide sequence of Psclone 51A and the amino acid sequence deduced for the open reading frame (ORF), is provided in figures 1, 2 and 3. The genetic sequence has been derived from cloned cDNA wherein the cDNA clones were derived from *Piscirickettsia salmonis* type strain (LF-89) messenger RNA (mRNA). The cloned material was sequenced in both directions from the 5' and 3' insertion sites using overlapping amplicons.

Veracity of the Psclone51A sequence was confirmed by Polymerase Chain Reaction(PCR) and Reverse-Transcriptase PCR (RT-PCR) of appropriate sized amplicons from *Piscirickettsia salmonis* infected Chinook salmon embryonic (CHSE-214) cell line and not from uninfected control material. Expression of the cloned sequence has yielded a protein of approximately 12 kDa.

The ORF of Psclone51A described in figure 1 does not have any significant homology at the nucleotide level with previous submissions to databases accessible by BLAST. At the protein level, a border line similarity with a hypothetical 21.5 kDa protein of *Escherichia coli* was found.

The ORF has commercial value for the following reasons:

There is sufficient reason to believe that the nucleotide corresponding amino acid sequence is of *Piscirickettsia salmonis* origin. As such its incorporation into nucleic acid vaccines may have an impact on the reduction of mortality of farmed Atlantic salmon caused by Piscirickettsia salmonis. Characterisation of the gene product will lead to the identification of key elements in pathogenesis of infection and to the design of more accurate diagnostic tests which will also aid in epidemiological studies documenting the dissemination of different strains of the disease.

The nucleotide sequence Psclone51A and associated derivatives thereof when translated into protein sequence being composed of either identical or equivalent amino acids, should induce a response by the hosts, immune system. This principle can be further expanded to use these proteins in diagnostics tests and vaccination procedures.

### Sequence ID No. 5 and 6 - p10.6

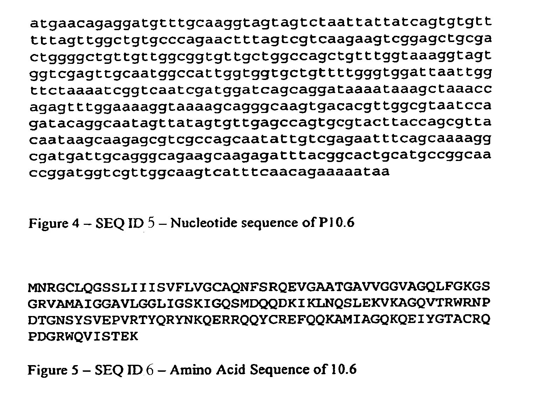

A detailed description of the invention, listing the exact nucleotide sequence of p10.6 and the amino acid sequence deduced for the open reading frame (ORF), is provided in figure 4 and figure 5 respectively.

The genetic sequence of Piscirickettsia salmonis (ATCC strain VR-1361), grown in CHSE-214 cells using homologous antisera from Piscirickettsia salmonis immunised rabbits, an immuno-reactive clone (0110-230 5) (plo.6) was identified from the expression library. This clone was sequenced and polymerase chain reaction (PCR) primers developed.

The Piscirickettsia salmonis origin of the p10.6 clone was confirmed through PCR amplification of Piscirickettsia salmonis and Chinook salmon embryonic cell line (CHSE-214) DNA using clone specific primers. Appropriately sized amplicons were amplified from the Piscirickettsia salmonis 8 DNA, but not from the CHSE-214 DNA confirming that the immuno-reactive clone was of Piscirickettsia salmonis origin and not from the host cell DNA.

The open reading frame (ORF) for the full gene was completed by inverse PCR from genomic Piscirickettsia salmonis DNA.

The ORF of p10.6 described in figure 4 does not have any significant homology at the nucleotide level with previous submissions to databases accessible by BLAST. The derived amino acid sequence, but not the nucleotide sequence, shows significant homology to the 17kDa antigen found in Rickettsia of the Spotted Fever Group, where it is considered a group specific, outer membrane protein.

### Sequence ID No 7 and 8 -IcmE (403)

A typical nucleic acid sequence is IcmE (403).

Peptide sequences transcribed or translated from the nucleic acid sequences of IcmE (403) may be incorporated into a vaccination strategy to induce an immune response to a surface antigen of Piscirickettsia salmonis itself.

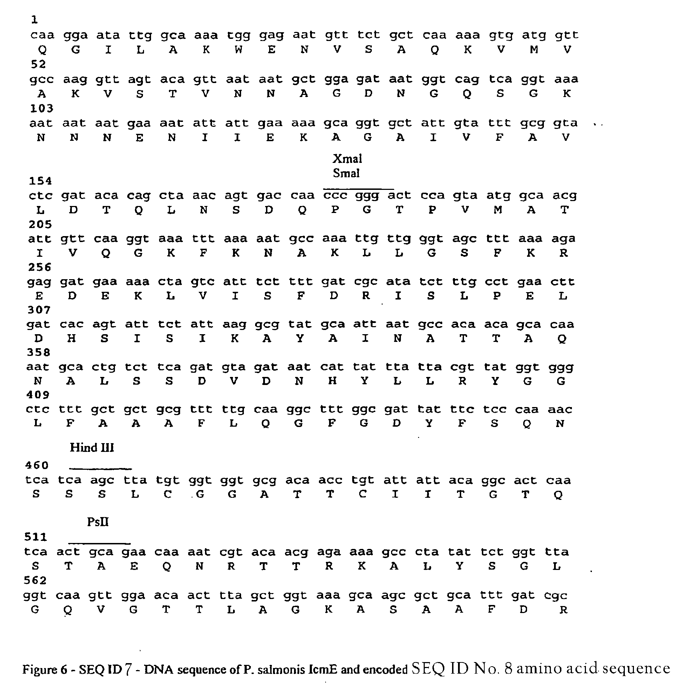

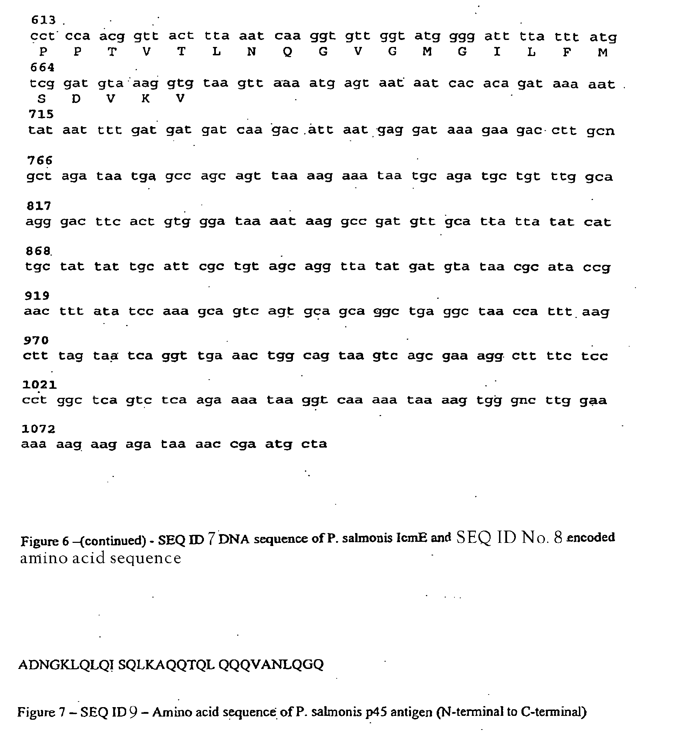

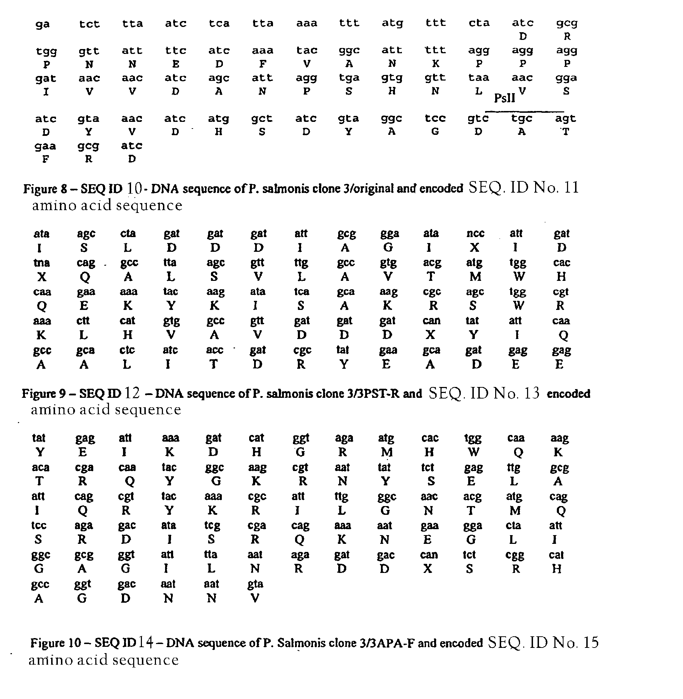

A detailed description of the invention, listing the exact nucleotide sequence of IcmE (403) and the amino acid sequence deduced for the open reading from (OFR), is provided in figure 6.

The genetic sequence has been derived from an inverse polymerase chain reaction (IPCR) product amplified from Piscirickettsia salmonis type strain (LF-89) genomic DNA (gDNA). The IPCR product was sequenced in both direction from the 5' and 3' sides using overlapping amplicons.

The protein encoded by the ORF of IcmE (403) has a 37% significant homology at the protein level to the IcmE protein of Legionella pneumophila when compared to previous submissions to databases accessible by BLAST.

### Sequence ID No. 9 -p45

A typical amino acid sequence is p45.

A nucleic acid sequence transcribing the amino acid sequence of p45 may be incorporated into a vaccination strategy to induce an immune response to a surface antigen against Piscirickettsia salmonis and thus to Piscirickettsia salmonis itself.

A detailed description of the invention, listing the exact amino acid sequence of a portion of the p45 major antigen, is provided in figure 7. The amino acid sequence has derived from microsequencing of a protein approximately 45 kDa found to be immunoreactive to rabbit anti-P salmonis antibodies. Moreover, p45 was found uniquely in Chinook salmon embryonic (SHSE-214) cells infected with Piscirickettsia salmonis and not in infected CHSE-214 cells.

The amino acid sequence of p45 has no significant homology to other bacterial proteins when compared to previous submissions to databases accessible by BLAST.

### Sequence ID No, 10,11,12,13,14 and 15 -clone 3

A typical nucleic acid sequence is clone3/original, clone 3/3PST-R and clone 3.3APA-F which corresponds to SEQ ID No.10, SEQ ID No.12 and SEQ ID No.14, respectively.

Peptide sequences transcribed or translated from the nucleic acid sequence of clone3/original, clone 3/3PST-R and clone 3.3APA-F may be incorporated into a vaccination strategy to induce an immune response to a surface antigen of Piscirickettsia salmonis genomic DNA (gDNA). The corresponding amino acid sequences to clone3/original, clone 3/3PST-R and clone 3.3APA-F are SEQ ID No.11, SEQ ID No.13 and SEQ ID No.15 respectively.

The proteins encoded by the ORF of clone3/original, clone3/3PST-R and clone3.3APA-F have respectively 40%, 38% and 34% significant homology at the protein level to different portion of the transposase protein of Vibrio anguillarum (gb AAA81776.1) when compared to previous submissions to databases accessible by BLAST.

### SEQ ID No 16, 17, 18, 19, 20, 21, 22 and 23 -clone 7

A typical nucleic acid sequence is clone 7/original, clone 7/7XbaR, clone7/7MunR and clone 7/7MunF which corresponds to SEQ ID No.16, SEQ ID No.18, SEQ ID No.20 and SEQ ID No.22, respectively.

Peptide sequences transcribed or translated from the nucleic acid sequence of clone7/original, clone7/7XbaR, clone7/7MunR, and clone 7/7MunF may be incorporated into a vaccination strategy to induce an immune response to a surface antigen of Piscirickettsia salmonis and thus to Piscirickettsia salmonis itself.

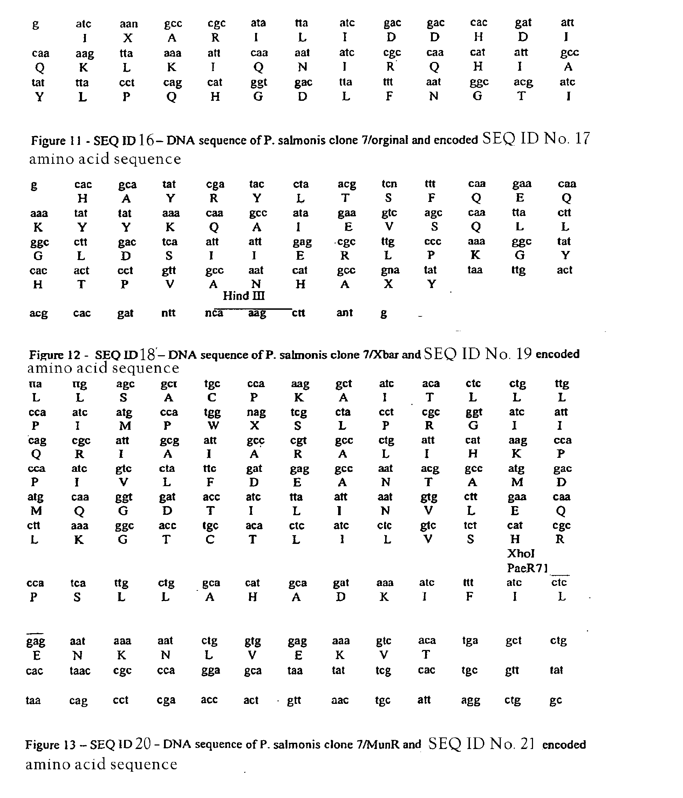

A detailed description of the invention, listing the extract nucleotide sequence of clone7/original, clone7/7XbaR, clone7/7MunR, and clone 7/7MunF and the amino acid sequence deduced for their open reading frames (ORF), is provided in figures 11, 12, 13 and 14 respectively.

The corresponding amino acid sequences to 7/original, clone 7/7XbaR, clone7/7MunR and clone 7/7MunF are SEQ ID No.17, SEQ ID No. 19, SEQ ID No.21 and SEQ ID No.23 respectively.

Some of the genetic sequences have been derived from an inverse polymerase chain reaction (IPCR) product amplified from Piscirickettsia salmonis genomic DNA (gDNA). The peptides encoded by the ORF of clone7/original, clone7/7XbaR, clone7/7MunR, and clone 7/7MunF have a 40% to 44% significant homology at the protein level to different portion of the ABC transporter ATP-binding protein of other bacterial species when compared to previous submissions to databases accessible by BLAST. There is sufficient reason to believe that the nucleotide and corresponding amino acid sequence are of Piscirickettsia salmonis origin. Also, part of the ORFs was found in an immuno-reactive clone of an expression library.

### Sequence ID No. 24,25,26,and 27 - clone 20

A typical nucleic acid sequence is clone20/original and clone 20/20VSPF corresponding to SEQ ID No.24 and SEQ ID No.26 respectively.

Peptide sequences transcribed or translated from the nucleic acid sequence of clone20/original, and clone20/20VSPF may be incorporated into a vaccination strategy to induce an immune response to a surface antigen of Piscirickettsia salmonis and thus to Piscirickettsia salmonis itself.

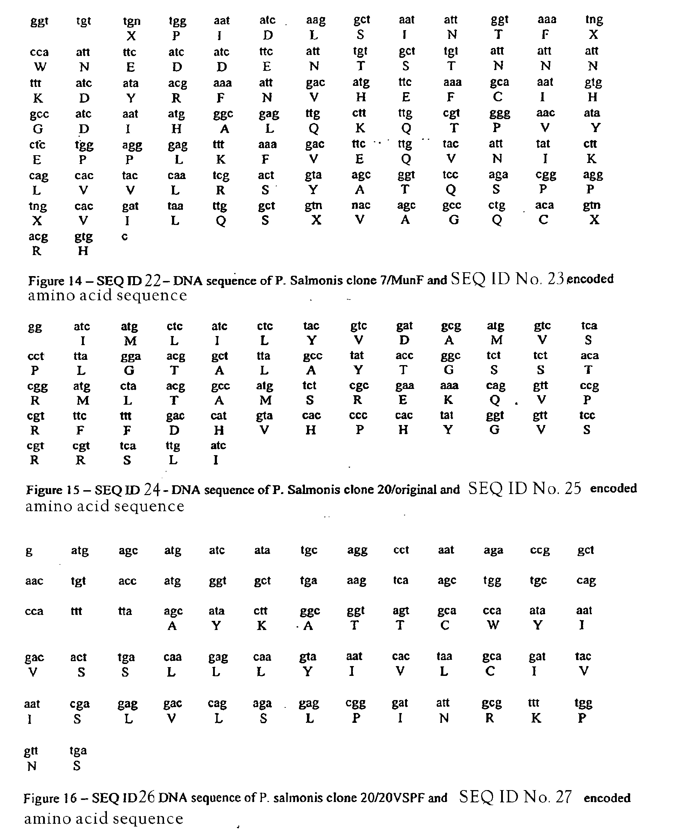

A detailed description of the invention, listing the exact nucleotide sequence of clone20/original, and clone20/20VSPF and the amino acid sequence deduced for their open reading frames (ORF), is provided in figure 15 and figure 16 respectively.

The corresponding amino acid sequences to clone20/original and clone 20/20VSPF are SEQ ID No.25 and SEQ ID No.27 respectively.

Some of the genetic sequences have been derived from an inverse polymerase chain reaction (IPCR) product amplified from Piscirickettsia salmonis genomic DNA (gDNA).

The peptides encoded by the ORF of clone20/original, and clone20/20VSPF have a 41% and 51% significant homology at the protein level to an amino acid transporter/permase protein of other organisms when compared to previous submissions to databases accessible by BLAST.

### Sequence ID 28 and 29 -clone 15

A typical nucleic acid sequence is clone15/original corresponding to SEQ ID No. 28.

Peptide sequences transcribed or translated from the nucleic acid sequence of clone15/original may be incorporated into a vaccination strategy to induce an immune response to a surface antigen of Piscirickettsia salmonis and thus to Piscirickettsia salmonis itself.

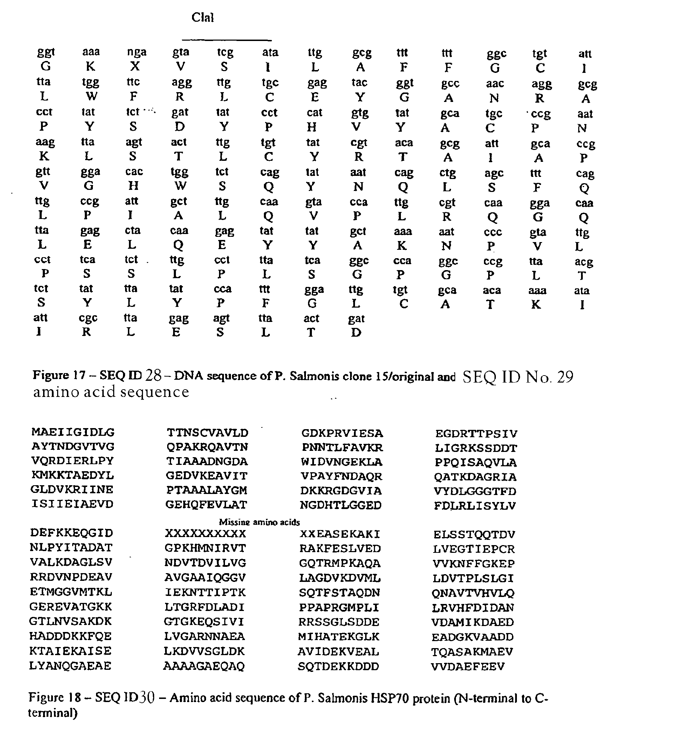

A detailed description of the invention, listing the exact nucleotide sequence of clone15/original and the amino acid sequence deduced for its open reading frames (ORF), is provided in figure 17. The amino acid sequence corresponding to clone 15/original is SEQ ID No.29.

The nucleotide sequence and the peptide encoded by the ORF of clone15/original have no significant homology to proteins of other bacterial species when compared to previous submissions to databases accessible by BLAST.

From the previous information there is sufficient reason to believe that the nucleotide and corresponding amino acid sequence are of Piscirickettsia salmonis origin. Also, the ORF was found in an immuno-reactive clone of an expression library.

### Sequence ID No.30 -Hsp 70

A typical amino acid sequence is Hsp 70 corresponding to SEQ ID No.30.

A nucleic acid sequence transcribing the amino acid sequence Hsp70 may be incorporated into a vaccination strategy to induce an immune response to a surface antigen of Piscirickettsia salmonis and thus to Piscirickettsia salmonis itself.

A detailed description of the invention, listing the exact amino acid sequence of the major portion of the HSP70 protein is provided in figure 18.

The amino acid sequence of Hsp70 has 70% significant homology at the protein level to Coxiella burnetti and Legionella pneumophila Hsp70 proteins when compared to previous submissions to databases accessible by BLAST.

The nucleic acid and amino acid sequences disclosed have commercial value for the following reasons:

From the previous information there is sufficient reason to believe that the nucleotide and corresponding amino acid sequences are of Piscirickettsia salmonis origin. Also, several of the ORF were found in immuno-reactive clones of an expression library. Together, this shows the potential of these ORFs to be incorporated into nucleic acid vaccines that may have an impact on the reduction of mortality of farmed salmonids due to *Rickettsial* Salmonid Septicaemia caused by *Piscirickettsia salmonis.*

PCR primers derived from the full ORF, used in touchdown PCR are capable of detecting *Piscirickettsia salmonis* in tissues from infected fish. This will lead to more accurate diagnostic tests to be used for clinical as well as epidemiological study purposes.

The nucleotide sequences associated derivatives thereof when translated into protein sequence being composed of either identical or equivalent amino acids, should induce a response by the hosts' immune system. This principle can be further expanded to use these proteins in diagnostic tests and nucleic acid vaccination procedures.

## Claims

1. A nucleic acid sequence derived from Piscirickettsia salmonis consisting of a sequence selected from the group consisting of SEQUENCE ID Numbers: 1, 3, 5, 7, 10, 12, 14, 16, 18, 20, 22, 24, 26 and 28 and synthetically prepared analogues thereof.

2. A nucleic acid sequence selected from the group according to Claim 1 which can be transcribed and/or translated to provide an amino acid sequence which corresponds to at least 70% of a surface antigen present on Piscirickettsia salmonis and induces an immune response.

3. A nucleic acid sequence according to claim 1 or claim 2 comprising the JcmE gene of Piscirickettsia salmonis type strain LF-89 corresponding to SEQ ID No. 7.

4. A nucleic acid sequence according to claim 1 or claim 2 comprising a cDNA sequence of Piscirickettsia salmonis type strain LF-89 which encodes a protein of approximately 12kDa.

5. A nucleic acid sequence according to claim 1 or claim 2 comprising a gene sequence of Piscirickettsia salmonis type strain LF-89 encoding an amino acid sequence showing significant homology to the 17kDa antigen found in Rickettsia of the Spotted Fever Group.

6. An amino acid sequence derived from the sequence of any of claims 1 to 5.

7. An amino acid sequence according to claim 6 which is translated from the sequence of any of claims 1 to 5.

8. An amino acid sequence derived from Piscirickettsia salmonis selected from the group consisting of SEQUENCE ID Numbers: 2, 4, 6, 8, 9, 11, 13, 15, 17, 19, 21, 23, 25, 27, 29, and 30, and synthetically prepared analogues thereof.

9. An amino acid sequence according to claim 8 which corresponds to at least 70% of a part of a surface antigen of Piscirickettsia salmonis and induces an immune response.

10. An amino acid sequence according to claim 8 or claim 9 selected from the sequence of p45 having SEQ. ID No. 9 or HSP70 having SEQ.ID No. 30 antigen from Piscirickettsia salmonis.

11. A diagnostic test kit comprising the amino acid sequence of any one of claims 6 to 10.

12. A nucleic acid PCR primer derived from the ORF of a nucleic acid sequence of any of claims 1 to 5 capable of detecting Piscirickettsia salmonis in tissues from infected fish.

13. Use of a nucleic acid PCR primer according to claim 12 in the preparation of a diagnostic test for detecting Piscirickettsia salmonis.

14. Use of an amino acid sequence according to any one of claims 6 to 10 in the preparation of a diagnostic test for detecting Piscirickettsia salmonis.

## Patentansprüche

1. Von Piscirickettsia salmonis abgeleitete Nukleinsäuresequenz, bestehend aus einer Sequenz, die aus der Gruppe der Sequenzen mit den Sequenz-ID-Nummern 1, 3, 5, 7, 10, 12, 14, 16, 18, 20, 22, 24, 26 und 28 sowie der synthetisch hergestellten Analoga dieser Sequenzen ausgewählt ist.

2. Nukleinsäuresequenz, ausgewählt aus der Gruppe nach Anspruch 1, die durch Transkription oder Translation in eine Aminosäuresequenz überführt werden kann, die zu mindestens 70 % einem auf Piscirickettsia salmonis vorliegenden Oberflächen-Antigen entspricht und eine Immunantwort auslöst.

3. Nukleinsäuresequenz nach Anspruch 1 oder 2, die das der SEQ ID No. 7 entsprechende IcmE-Gen des Piscirickettsia salmonis-Typstamms LF-89 enthält.

4. Nukleinsäuresequenz nach Anspruch 1 oder 2, die eine cDNA-Sequenz des Piscirickettsia salmonis-Typstamms LF-89 umfasst, welche für ein Protein von ungefähr 12 kDa codiert.

5. Nukleinsäuresequenz nach Anspruch 1 oder 2, die eine Gensequenz des Piscirickettsia salmonis-Typstamms LF-89 umfasst, welche für eine Aminosäuresequenz codiert, die eine signifikante Homologie zu dem in Rickettsien der Fleckfiebergruppe gefundenen 17 kDa-Antigen aufweist.

6. Aminosäuresequenz, abgeleitet von der Sequenz eines der Ansprüche 1 bis 5.

7. Aminosäuresequenz nach Anspruch 6, die durch Translation der Sequenz eines der Ansprüche 1 bis 5 erhalten wird.

8. Von Piscirickettsia salmonis abgeleitete Aminosäuresequenz, ausgewählt aus der Gruppe der Sequenzen mit den Sequenz-ID-Nummern 2, 4, 6, 8, 9, 11, 13, 15, 17, 19, 21, 23, 25, 27, 29 und 30 sowie der synthetisch hergestellten Analoga dieser Sequenzen.

9. Aminosäuresequenz nach Anspruch 8, die zu mindestens 70 % einem Teil eines Oberflächen-Antigens von Piscirickettsia salmonis entspricht und eine Immunantwort auslöst.

10. Aminosäuresequenz nach Anspruch 8 oder 9, ausgewählt unter der Sequenz des Piscirickettsia salmonis-Antigens p45 mit der SEQ ID No. 9 und der Sequenz des Piscirickettsia salmonis-Antigens HSP70 mit der SEQ ID No. 30.

11. Diagnostisches Test-Kit, das die Aminosäuresequenz eines der Ansprüche 6 bis 10 umfasst.

12. Nukleinsäure-PCR-Primer, abgeleitet vom ORF einer Nukleinsäuresequenz eines der Ansprüche 1 bis 5, der in der Lage ist, Piscirickettsia salmonis in Geweben infizierter Fische zu erkennen.

13. Verwendung eines Nukleinsäure-PCR-Primers nach Anspruch 12 zur Entwicklung eines diagnostischen Tests zur Erkennung von Piscirickettsia salmonis.

14. Verwendung einer Aminosäuresequenz nach einem der Ansprüche 6 bis 10 zur Entwicklung eines diagnostischen Tests zur Erkennung von Piscirickettsia salmonis.

## Revendications

1. Séquence d'acide nucléique dérivée de *Piscirickettsia salmonis* composée d'une séquence sélectionnée à partir du groupe comprenant les numéros de SEQUENCE ID : 1, 3, 5, 7, 10, 12, 14, 16, 18, 20, 22, 24, 26 et 28 et ses analogues préparés de façon synthétique.

2. Séquence d'acide nucléique sélectionnée à partir du groupe selon la revendication 1 qui peut être transcrite et/ou transcodée de façon à procurer une séquence d'acide aminé qui correspond à au moins 70% d'un antigène de surface présent sur *Piscirickettsia salnionis* et à induire une réponse immunitaire.

3. Séquence d'acide nucléique selon la revendication 1 ou 2 comprenant le gène IcmE de la souche LF-89 du type *Piscirickettsia salmonis* qui correspond à la SEQ ID No. 7.

4. Séquence d'acide nucléique selon la revendication 1 ou 2 comprenant une séquence d'ADNc de la souche LF-89 du type *Piscirickettsia salmonis* qui code pour une protéine d'environ 12kDa.

5. Séquence d'acide nucléique selon la revendication 1 ou 2 comprenant une séquence génique de la souche LF-89 du type *Piscirickettsia salmonis* qui code pour une séquence d'acide aminé faisant preuve d'une homologie significative avec l'antigène 17kDa trouvé dans le Rickettsia du groupe fièvre exanthématique.

6. Séquence d'acide aminé dérivée d'une séquence selon l'une quelconque des revendications 1 à 5.

7. Séquence d'acide aminé selon la revendication 6 qui est transcodée à partir de la séquence selon l'une quelconque des revendications 1 à 5.

8. Séquence d'acide aminé dérivée de *Piscirickettsia salmonis* sélectionnée à partir du groupe comprenant les numéros de SEQUENCE ID: 2, 4, 6, 8, 9, 11, 13, 15, 17, 19, 21, 23, 25, 27, 29 et 30 et ses analogues préparés de façon synthétique.

9. Séquence d'acide aminé selon la revendication 8 qui correspond à au moins 70% d'une partie d'un antigène de surface présent sur *Piscirickettsia salmonis* et à induire une réponse immunitaire.

10. Séquence d'acide aminé selon la revendication 8 ou 9 sélectionné à partir de la séquence p45 possédant la SEQ. ID No. 9 ou à partir de HSP70 possédant la SEQ. ID No. 30 provenant de *Piscirickettsia salmonis.*

11. Kit pour test diagnostique comprenant la séquence d'acide aminé selon l'une quelconque des revendications 6 à 10.

12. Amorce PCR de l'acide nucléique dérivée de l'ORF d'une séquence d'acide nucléique selon l'une quelconque des revendications 1 à 5 capable de détecter *Piscirickettsia salmonis* dans les tissus de poissons infectés.

13. Utilisation d'une amorce PCR de l'acide nucléique selon la revendication 12 dans la préparation d'un test diagnostique destiné à détecter *Piscirickettsia salmonis.*

14. Utilisation d'une séquence d'acide aminé selon l'une quelconque des revendications 6 à 10 dans la préparation d'un test diagnostique destiné à détecter *Piscirickettsia salmonis.*
